# EUROPEAN PATENT APPLICATION

(11) **EP 4 497 748 A1**
(43) Date of publication of application: **29.01.2025**
(21) Application number: 23187383.7
(22) Date of filing: 24.07.2023
(51) Int. Cl.: C07D 311/72, C07C 229/06, A23L 33/15, A23L 33/175, A23K 20/142, A23K 20/174

(54) **IMPROVED MULTICOMPONENT CRYSTALS OF TOCOPHEROL MIXTURES AND PROCESS OF MAKING**

(71) Applicant: BASF SE, 67056 Ludwigshafen am Rhein (DE)
(72) Inventor: Viertelhaus, Martin, 67056 Ludwigshafen am Rhein (DE); Hellmann, Rolf, 67056 Ludwigshafen am Rhein (DE); Hermannsdoerfer, Dirk, 67056 Ludwigshafen am Rhein (DE); Bruhns, Stefan, 67056 Ludwigshafen am Rhein (DE); Aponte, Raphael, 67056 Ludwigshafen am Rhein (DE)
(74) Representative: BASF IP Association

(57) **Abstract**

Multicomponent crystal comprising
a) an active, said active being a mixture of at least two molecules of formula (1) 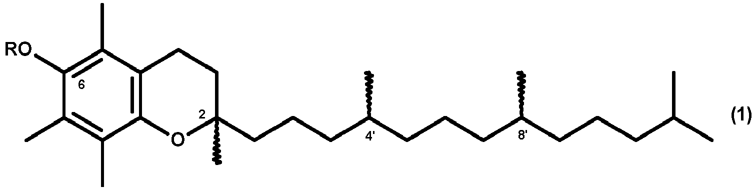 with R being at least one selected from the group consisting of H, COCH₃, COCH₂CH₃, CO(CH₂)₁₄CH₃, CO(CH₂)₁₆CH₃,
- with the methyl groups in position 2, 4', 8' respectively having a S-configuration or a R-configuration,
and

b) a crystallizing agent (2), said crystallizing agent (2) exhibiting the property of
- being suitable to form ions and/or
- containing a H-bond donor and/or
- containing a H-bond acceptor and/or
- being suitable to form a zwitterionic structure and/or
- having a molecular weight, which ranges from 100 g/mol to 135 g/mol,
with the proviso that the combination of proline as crystallizing agent (2) and tocopherol as active is excluded from the teaching of claim 1.

## Description

This invention relates to improved multicomponent crystals of α-tocopherol mixtures as well as to a process of making same. A further aspect of the invention covers a composition comprising said multicomponent crystals as well as the use of said multicomponent crystals and said composition.

Synthetic α-tocopherol or syn α-tocopherol, also referred to as DL-α-tocopherol or D/L α-tocopherol is an isomeric mixture comprising at least two stereoisomers. It can also comprise three stereoisomers, four stereoisomers, five stereoisomers, six stereoisomers, seven stereoisomers and in most cases eight stereoisomers. It can be defined by formula (3) 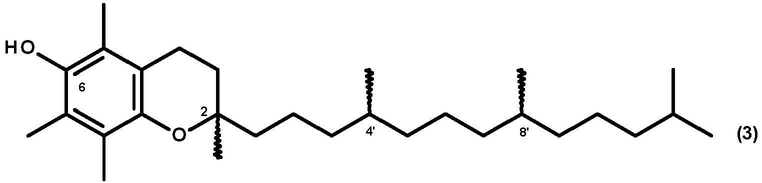 with the methyl groups in position 2, 4', 8' respectively having a S-configuration or a R-configuration,

In case of all eight stereoisomers being present, synthetic α-tocopherol or DL-α-tocopherol is also named all-rac α-tocopherol. All-rac α-tocopherol has three stereocenters viz. at position 2, 4' and 8'as shown in formula (3) and thus it can form the eight stereoisomers having the following conformation: RRR, RRS, RSS, SSS, RSR, SRS, SRR and SSR.

DL-α-tocopherol as well as all-rac α-tocopherol are synthetically produced mixtures of Vitamin E active compounds or actives of formula (3). They are highly viscous oils, which are sensitive to oxidation and thus not stable for extended storage. Taking into account this sensitivity towards oxygen, stemming from the OH-group in position 6 of the aromatic ring of DL-α-tocopherol as well as all-rac α-tocopherol, both shown in formula (3), successful attempts were made to protect said OH group of said actives against oxidation by means of esterification.

Beside the actives DL-α-tocopherol as well as all-rac α-tocopherol, both shown in formula (3), the acetate, propionate, palmitate and stearate of DL-α-tocopherol as well as of all-rac α-tocopherol became important feed and food supplements and pharmaceutical ingredients. Thus, commercial important compound mixtures exhibiting Vitamin E activity are not only the compound mixtures of formula (3) but rather the mixtures of the larger group of DL-α-tocopherol, all-rac α-tocopherol and their respective esters, all of which are grouped under formula (1) 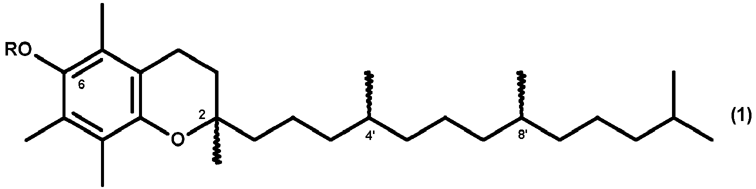 with R being at least one selected from the group consisting of H, COCH₃, COCH₂CH₃, CO(CH₂)₁₄CH₃, CO(CH₂)₁₆CH₃ and with the methyl groups in position 2, 4', 8' respectively having a S-configuration or a R-configuration.

However, also the actives or active mixtures of formula (1) are either highly viscous oils difficult to handle and to formulate or fragile solids. All of them are light-sensitive and degrade upon application of elevated temperatures. They do not exhibit characteristics of a fine powder and unfavorably tend for clumping or caking. In order to preserve the mixtures of formula (1) from oxidation or degradation by light or high temperature and to achieve non-hygroscopic defined solids, sophisticated formulation technology is needed to produce a lasting free flowing powder. To obtain good formulation properties carbohydrates, colloids and/or silica are generally used as adjuvants in an expensive spray drying or beadlet process.

An attempt to overcome the previously mentioned drawbacks was made by Mei and coworkers in EP 3 733 657 A1. They produced crystalline or partial crystalline "co-crystals" of tocopherol and proline (cf. para. 15).

In this reference `657 tocopherol is understood to mean: "Tocopherols include natural tocopherol and synthetic tocopherol. Natural tocopherol comprises two types of tocopherols and tocotrienols, and comprises 8 kinds of compounds, namely α-, β-, γ-, δ-tocopherols and α-, β-, γ-, δ-tocotrienols. Synthetic tocopherol refers to α-tocopherol, and has 8 optical isomers. Among them, D-α-tocopherol is a form of tocopherol which is most widely distributed in nature, most abundant, and has the highest activity." (cf. para. 0002) and "The tocopherol comprises natural tocopherol and synthetic tocopherol, the natural tocopherol comprises α-, β-, γ-, δ-tocopherols and α-, β-, γ-, δ-tocotrienols; the synthetic tocopherol comprises DL-α-tocopherols." (cf. claim 2).

Likewise, a definition is given for proline, which reads: "Preferably, the proline is D-proline, or L-proline, or a racemic mixture of the both." (cf. para 7 of `657) and "The proline comprises D-proline, or L-proline, or a racemic mixture of the both." (cf. claim 2).

Upon reworking the examples of `657, we found, that the onset temperatures Tₒ, the peak temperatures Tₚ and the transition enthalpy ΔH all measured by differential scanning calorimetry were low (cf. comparative examples below). Likewise the temperature of decomposition T_{d} as measured by differential gravimetric analysis (TGA) is reduced. This indicates crystallinity in `657 being moderate or only partially developed and samples being heterogeneous and partially amorph or still containing not crystallized matter.

In Int. J. Pharm. 592 (2021) 120057, hereinafter '057, Mei and coworkers study co-crystals or partially crystalline co-crystals formed from the single molecule D-α-tocopherol, also named RRR-α-tocopherol and either 1 ,2-di (4-pyridyl)ethylene (DPE) or L-proline or betaine. This is to say, that mixtures like DL-α-tocopherol or all-rac α-tocopherol are not assayed for co-crystal formation, let alone mixtures of formula (1).

We reproduced the preparation indicated under 2.4. of '057 comprising D-α-tocopherol and betaine (cf. comparative examples below) and could only establish the partial formation of co-crystals.

The invention intends to overcome the drawbacks of the prior art. In particular, it is an object of the invention to obtain a more homogeneous or higher structured and thus more stable multi-component crystal. Another object of the invention is to obtain this more stable multicomponent crystal not from D-α-tocopherol but from a mixture of at least two molecules of formula (1) or of formula (3). Yet another inventive object is to obtain a multicomponent crystal which remains stable at higher temperatures than in those disclosed the prior art. A further inventive objective is to obtain a multicomponent crystal, which is free flowable and does not clot. It shall not tend to be hygroscopic, even after storage periods over weeks and months. Another object is to provide a multicomponent crystal, which can be readily formulated into a composition of the invention. Yet another object is to obtain a multicomponent crystal, in which the bioavailability is as in the mixture of formula (1) or of formula (3) prior to crystallization or is only slightly reduced with respect to the uncrystallized mixture of formula (1) or of formula (3).

A further object of the invention is to provide a process for obtaining the inventive multicomponent crystal. Said process is to be straight forward, shall avoid harmful operation steps or compounds and shall be inexpensive. The way of conducting said process shall not be excessively time-consuming and easy to realize. Furthermore, said process shall ensure a huge amount of active to become part of the multicomponent crystal. It shall avoid to the utmost extent having non-crystallized matter being part of the multicomponent crystal.

Yet another object of the invention is to provide a composition comprising the multicomponent crystal. Said composition shall be timely obtained. It shall exhibit at least the performance characteristics of state-of-the-art formulations of DL-α-tocopherol or of all-rac α-tocopherol and even perform better. Said composition shall have the same or a better bioavailability as prior art DL-α-tocopherol or of all-rac α-tocopherol compositions. It shall exhibit at least identical and even better rheology characteristics/flowability than art prior art DL-α-tocopherol or of all-rac α-tocopherol compositions. Another object of the invention shall be to design said composition such that it can be made with low cost.

Another object of the invention is to provide uses for the inventive multicomponent crystal, its process of making and for the compositions comprising the multicomponent crystal.

All these objects are fulfilled by a multicomponent crystal comprising
a) an active, said active being a mixture of at least two molecules of formula (1) 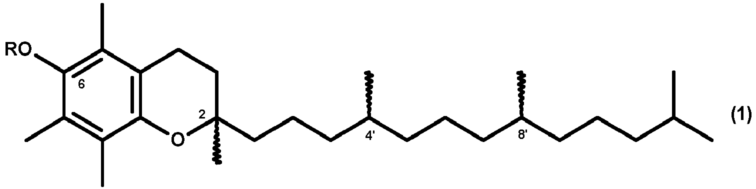 with R being at least one selected from the group consisting of H, COCH₃, COCH₂CH₃, CO(CH₂)₁₄CH₃, CO(CH₂)₁₆CH₃,
   - with the methyl groups in position 2, 4', 8' respectively having a S-configuration or a R-configuration,
   and
b) a crystallizing agent (2), said crystallizing agent (2) exhibiting the property of
   - being suitable to form ions and/or
   - containing a H-bond donor and/or
   - containing a H-bond acceptor and/or
   - being suitable to form a zwitterionic structure and/or
   - having a molecular weight, which ranges from 100 g/mol to 135 g/mo,
with the proviso that the combination of proline as crystallizing agent (2) and tocopherol as active is excluded from the teaching of this embodiment.

Said multicomponent crystal comprising as active a mixture of at least two molecules of formula (1) and a crystallizing agent (2), which exhibits the property of being suitable to form ions and/or contains a H-bond donor and/or contains a H-bond acceptor and/or being suitable to form a zwitterionic structure and/or having a molecular weight, which ranges from 100 g/mol to 135 g/mol, with the proviso that the combination of proline as crystallizing agent (2) and tocopherol as active is excluded from the teaching of this embodiment, gives rise to advantages in comparison to neat DL-α-tocopherol or DL-α-tocopherol L-proline co-crystal with respect to flowability, stability, hygroscopicity, storage stability, bio-availability, purity, purification. This means that the new multicomponent crystal in solid form is stable at higher temperatures compared to those of the prior art. It is obtained as fines. It has for example better flowability without clotting, lower hygroscopicity even after storage periods over weeks and months, better storage stability, higher purity and better purification properties. The inventive multicomponent crystal does not require any further purification and can be directly formulated into a composition. Compared to compositions of the prior art containing an active of formula (1) or of formula (3) the inventive multicomponent crystal preferably exhibits higher bioavailability and bioavailability with less variability. The bioavailability of the mixture of formula (1) or of formula (3) in the new multicomponent crystal is higher, comparable or only slightly lower than this one of the uncrystallized mixture of formula (1) or of formula (3).

Said multicomponent crystal is also suited to be employed as intermediate or starting material to produce a pure active out of a mixture of a multitude of compounds..

All this is due to the higher degree of crystallinity, to a more homogeneous or higher structured and thus more stable multicomponent crystal, as can be deduced from the respective onset temperature Tₒ, the respective peak temperature Tₚ and from the respective enthalpy ΔH as measured by DSC and shown below, and which are respectively higher than for neat DL-α-tocopherol or for co-crystals comprising a combination of proline as crystallizing agent (2) and tocopherol as active.

This is astonishing, since the active of the invention is not a single distinct compound but a mixture of several distinct compounds and/or a mixture of several stereoisomers of a distinct compound. Distinct moieties of the molecules in such mixtures, due to their respective isomeric structure, have different special orientations. They are thus not prone to readily arrange in a highly ordered structure with the crystallizing agent (2) as achieved. One would rather expect to obtain an amorphous precipitate if at all or a multicomponent crystal of only one diastereomer, and not a well defined multicomponent crystal as shown in the PXRD-diagrams infra.

Multicomponent crystal as understood under this invention means a crystal, viz a highly ordered structure comprising an active and a crystallizing agent (2). Crystalline parts of said crystal can have different orientations into one and the same particle.. There can be also parts, which still have an amorphous character, however the degree of crystallinity in the multicomponent crystal is higher than in the co-crystals of the prior art. The word "multicomponent crystal" and "multi-component crystals" are understood to have the same meaning and they are reciprocally used in this disclosure.

However, crystals formed from proline and tocopherol do not exhibit the increased stability, the better rheological properties and the more pronounced crystallinity of the multicomponent crystal.

Tocopherol as understood within the phrasing "with the proviso that the combination of proline as crystallizing agent (2) and tocopherol as active is excluded from the teaching of this embodiment." means natural tocopherol and synthetic tocopherol. Natural tocopherol comprises two types of tocopherols and tocotrienols, and comprises 8 kinds of compounds, namely α-, β-, γ-, δ-tocopherols and α-, β-, γ-, δ-tocotrienols. Synthetic tocopherol refers to α-tocopherol, and has 8 optical isomers, viz. at least one, two, three, four, five, six, seven or eight of RRR, RRS, RSS, SSS, RSR, SRS, SRR and SSR.

Proline as understood within the phrasing "with the proviso that the combination of proline as crystallizing agent (2) and tocopherol as active is excluded from the teaching of this embodiment." means proline to comprise D-proline, or L-proline, or a racemic mixture of the both.

An active of the invention in any case is a mixture of at least two molecules and not a single compound like e.g. D-α-tocopherol. Actives of the invention can be mixtures of different molecules having the same stereochemistry, like e.g. D-α-tocopherol and D-α-tocopherol acetate. Also at least one of propionate, palmitate and stearate of D-α-tocopherol combined with at least one of D-α-tocopherol or D-α-tocopherol acetate can be an active Every permutation of the previously mentioned molecules is possible.

Actives can also be mixtures of different stereoisomers of a molecule with a defined molecular weight, like e.g. DL-α-tocopherol having a variable amount of stereoisomers or of all-rac α-tocopherol having eight stereoisomers.

An active can also be a mixture of at least one distinct stereoisomer of a molecule with a defined molecular weight combined with mixtures of different molecules having the same stereochemistry. An example would be D-α-tocopherol palmitate combined with D-α-tocopherol and D-α-tocopherol stearate.

Finally, an active can be a mixture of distinct stereoisomers of a molecule with at least one molecule having a defined molecular weight. An example would be RRR-tocopherol acetate, RSS-tocopherol acetate and D-α-tocopherol stearate.

A crystallizing agent (2) is any compound, which meets at least one, preferably all of the conditions, being suitable to form ions and/or containing a H-bond donor and/or containing a H-bond acceptor and/or being suitable to form a zwitterionic structure and/or having a molecular weight, which ranges from 100 g/mol to 135 g/mol. Preferably a crystallizing agent (2) is any dietary acceptable compound, adapted to form ions and/or contains a H-bond donor and/or contains a H-bond acceptor and/or being suitable to form a zwitterionic structure and/or having a molecular weight, which ranges from 100 g/mol to 135 g/mol. Exemplary co-crystallizing agents (2) are nicotinic acid, nicotinamide, succinic acid, malic acid.A highly preferred crystallizing agent is betaine.

In a further embodiment of the invention the multicomponent crystal defines
- the active being a mixture of at least three molecules of formula (1), preferably of at least four molecules of formula (1), further preferred of at least five molecules of formula (1), yet further preferred of at least six molecules of formula (1), still further preferred of at least seven molecules of formula (1) and mostly preferred of at least eight molecules of formula (1),
   and/or
- R in formula (1) being at least one selected from the group consisting of H and COCH₃,
   and/or
- the crystallizing agent (2) being betaine of formula (2) 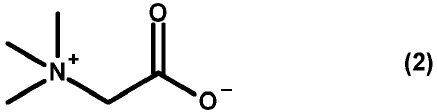 in its ionic form, which it adopts during crystallization.

Upon using betaine as crystallizing agent of formula (2) the advantages of claim 1, viz. better flowability, stability, hygroscopicity, storage stability, bioavailability, purity, purification behavior are even improved, as can be seen below. This means that the such solid form of multicomponent crystal has better flowability, lower hygroscopicity, better storage stability, higher bioavailability, bioavailability with less variability, higher purity and better purification properties.

A further developed embodiment of the multicomponent crystal of the invention
- the active being D/L-α-tocopherol of formula (3) 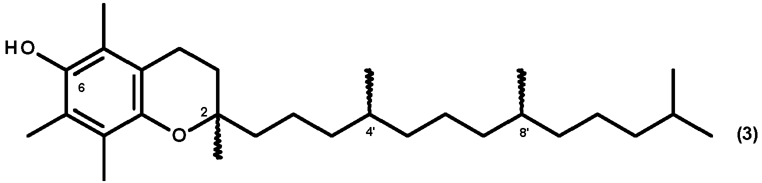 with the methyl groups in position 2, 4', 8' respectively having a S-configuration or a R-configuration,
   and/or
- the crystallizing agent (2) being betaine of formula (2) 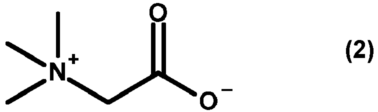 in its ionic form, which it adopts during crystallization.

The stabilization of D/L-α-tocopherol or of all-rac α-tocopherol by means of esterification in order to reduce their sensitivity to oxidation also reduces to some extent their capacity of acting as antioxidant and their bioavailability. This somewhat small, however existing disadvantage can be overcome by using D/L-tocopherol of formula (3) synonymous to DL-tocopherol of formula (3), since in these entities, there is free access to the OH-group in position 6 of formula (3). This however is not detrimental, since the molecule of formula (3) is part of the multicomponent crystal.

Provided one uses an excess of active, not all of it can be incorporated into the multicomponent crystal. A deficiency of active on the other hand would reduce its deliverable quantity e.g. in a dietary supplement and thus reduces the amount to be bioavailable accessible for the body. This can be avoided with a multicomponent crystal of the invention exhibiting the molar ratio of the active to the crystallizing agent (2) to range from 3:1 to 1:3, preferably from 2.1:1 to 1.9:1 or from 2:1 to 1:2 including a molar ratio of 1:1 and further preferred being 2:1.

The multicomponent crystal of the invention has a powder x-ray diffraction pattern (PXRD pattern) with at least one characteristic peak expressed in °2θ ± 0.2 °2θ (CuKa radiation), which is selected from the peaks located at 5.5, 7.4, 9.2, 12.9, 16.7 and 20.4, preferably it has a powder x-ray diffraction pattern (PXRD pattern) with at least three characteristic peaks expressed in °2θ ± 0.2 °2θ (CuKa radiation), which are selected from the peaks located at 5.5, 7.4, 9.2, 12.9, 16.7 and 20.4, further preferred it has a powder x-ray diffraction pattern (PXRD pattern) with the following characteristic peaks expressed in °2θ ± 0. 2 °2θ (CuKa radiation) and located at 5.5, 7.4, 9.2, 12.9, 16.7 and 20.4.

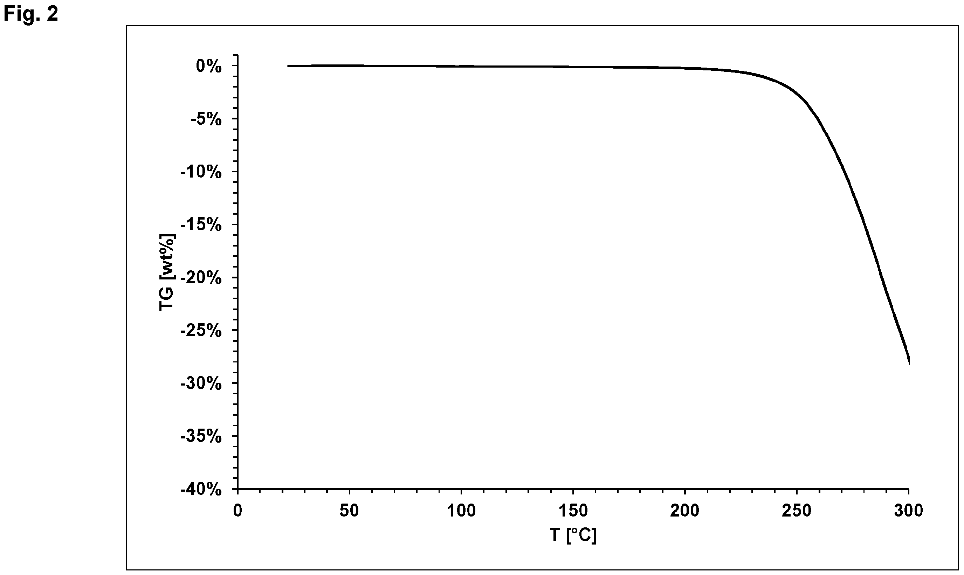

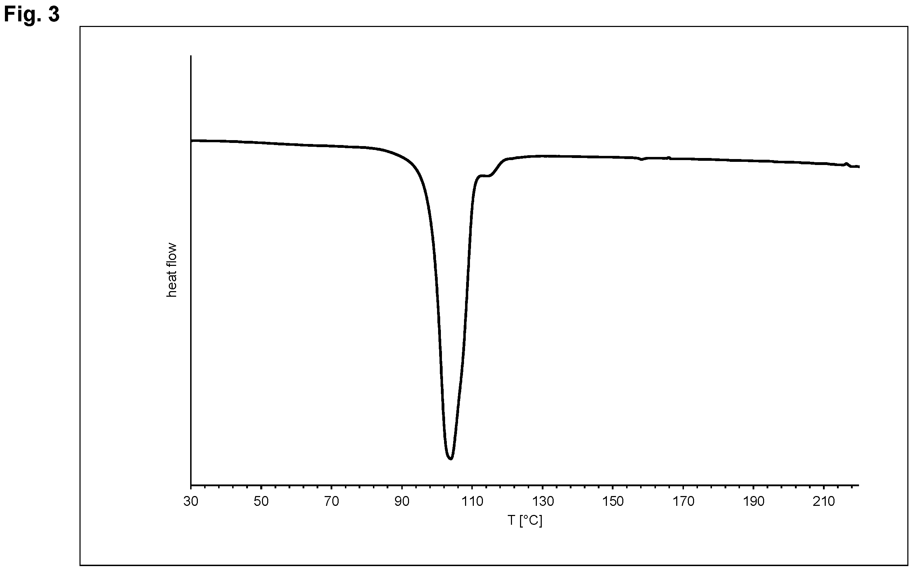

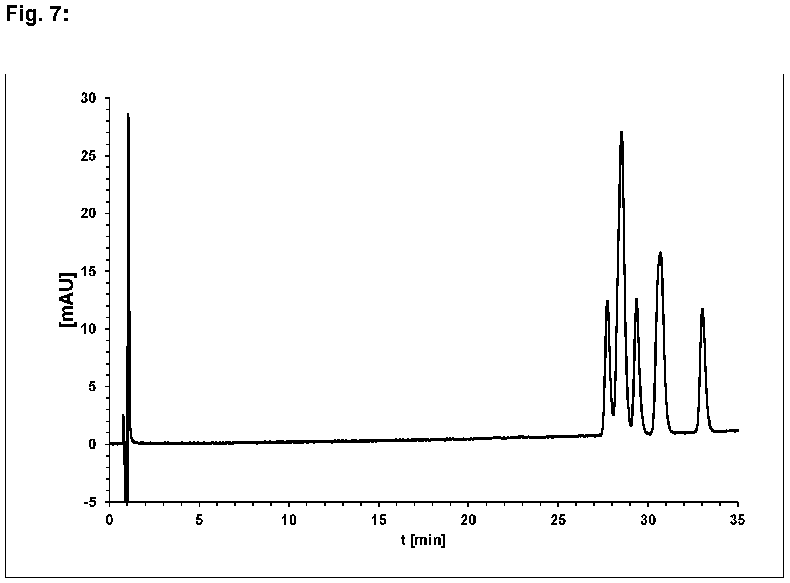

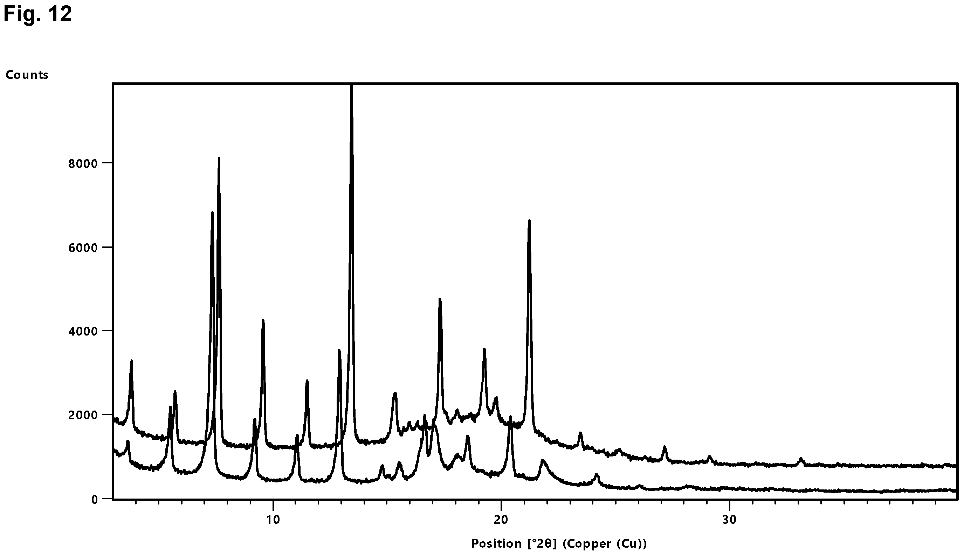

One observes the peaks of the highly crystalline inventive multicomponent crystal comprising the active and betaine to be different compared to the co-crystal of D-α-tocopherol and betaine disclosed in comparative example 13, as shown in Fig. 12. This is also backed by the thermogravimetric analysis (TGA) results of example 1 and Fig. 2 showing a high temperature of decomposition T_{D} of 200 °C and by the high onset temperature T_{O} of 98 °C, the high peak temperature T_{P} of 103 °C and the high phase transition enthalpy ΔH of 82 J/g as given in example 1 and Fig. 3, all of which indicating a well packed highly structured multicomponent crystal, which can only be disassembled at high temperatures and with an elevated amount of energy. As shown by HPLC analysis the multicomponent crystal comprises all eight stereoisomers in the same ratio as observed in all-rac α-tocopherol (cf. Fig. 7).

A further advanced embodiment of the inventive multicomponent crystal has a powder x-ray diffraction pattern (PXRD pattern) with at least one characteristic peak expressed in °2θ ± 0.2 °2θ (CuKa radiation), which is selected from the peaks located at 3,7, 5.5, 7.4, 9.2, 11,1 12.9, 14,8, 15,6, 16.7, 17,0, 18,1, 18,5, 20.4, 21,8 and 24,2, preferably it has a powder x-ray diffraction pattern (PXRD pattern) with at least three characteristic peaks expressed in °2θ ± 0.2 °2θ (CuKa radiation), which are selected from the peaks located at 3.7, 5.5, 7.4, 9.2, 11.1 12.9, 14.8, 15.6, 16.7, 17.0, 18.1, 18.5, 20.4, 21.8 and 24.2, further preferred it has a powder x-ray diffraction pattern (PXRD pattern) with the following characteristic peaks expressed in °2θ ± 0.2 °2θ (CuKa radiation) and located at 3.7, 5.5, 7.4, 9.2, 11.1 12.9, 14.8, 15.6, 16.7, 17,0, 18.1, 18,5, 20.4, 21,8 and 24,2.

One observes the peaks of the highly crystalline inventive multicomponent crystal comprising D/L- α-tocopherol and betaine to be different compared to the co-crystal of D-α-tocopherol and betaine disclosed in comparative example 13, as shown in Fig. 12. This is also backed by the thermogravimetric analysis (TGA) results of example 1 and Fig. 2 showing a high temperature of decomposition T_{D} of 200 °C and by the high onset temperature T_{O} of 98 °C, the high peak temperature T_{P} of 103 °C and the high phase transition enthalpy ΔH of 82 J/g as given in example 1 and Fig. 3, all of which indicating a well packed highly structured multicomponent crystal, which can only be disassembled at high temperatures and with an elevated amount of energy. Likewise, the total recovery and same repartition of different stereoisomers in the multicomponent crystal when compared to neat D/L- α-tocopherol, reveals a highly ordered and not easily dissolvable structure of the inventive multicomponent crystal. As shown by HPLC analysis the multicomponent crystal comprises all eight stereoisomers in the same ratio as observed in all-rac α-tocopherol (cf. Fig. 7).

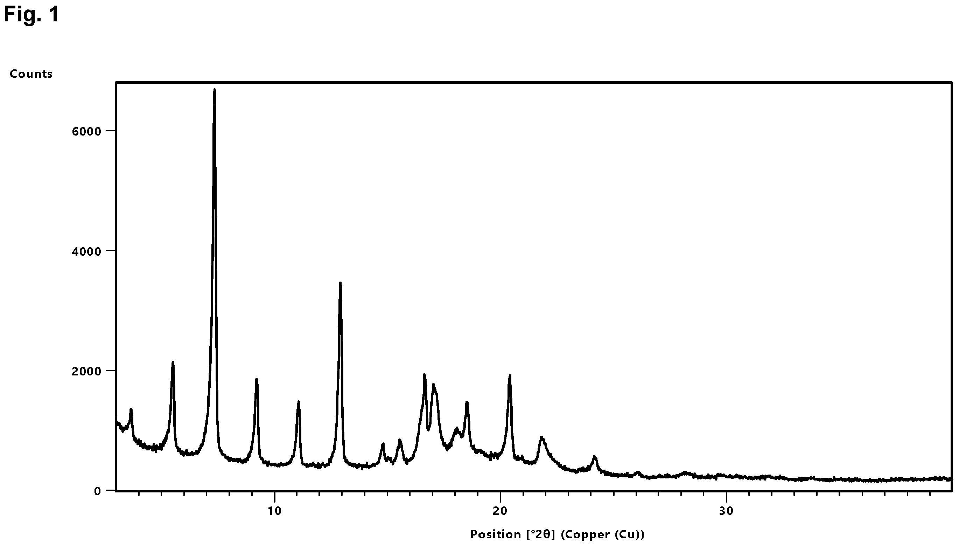

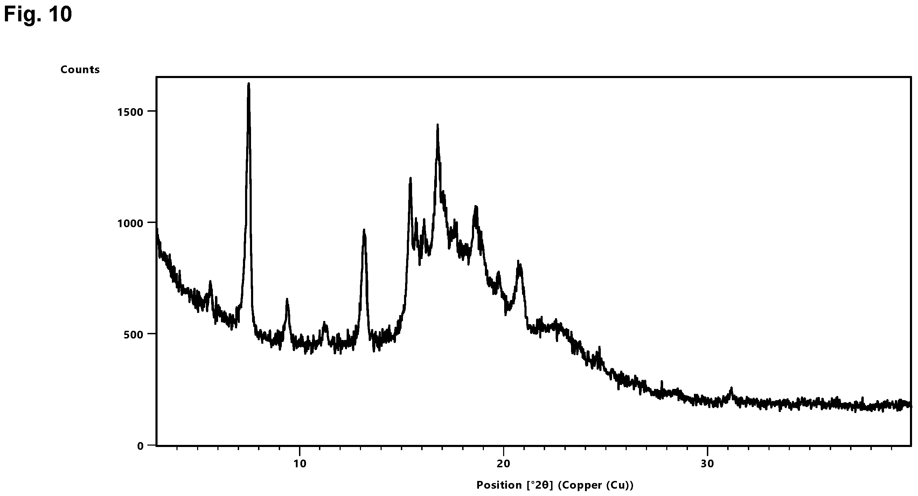

Co-crystals of the prior art still comprise as non-crystallized matter free D/L-α-tocopherol as shown in comparative example 9. Non crystallized matter can be any active of formula (1) or of formula (3), which does not get appropriately located in the ordered structure of a crystal or sticks in an amorphous way either to its surface and/or in holes, gaps or interstitials of a less ordered or partially amorphous crystal. This likewise contributes to make the crystal less homogeneous and less structured as can be seen in the powder x-ray diffraction diagrams (PXRD) of Fig. 10 compared to Fig. 1 and Fig. 4, Fig. 10 exhibiting a greater background especially in the middle of the diagram.

In contrast, the multicomponent crystal of the invention comprises less than 45 w% of not crystallized matter, preferably less than 30 w%, further preferred less than 20 w%, still further preferred less than 10 w% and highly preferred less than 5 w% including 1 w% and 0 w%.

As already mentioned, higher crystallinity of this embodiment provides better stability, flowability and hygroscopicity.

As previously mentioned, the inventive multicomponent crystal presents a highly ordered structure and only a small to no amount of excess or not crystallized active is present. Only then, elevated onset temperatures Tₒ, peak temperatures Tₚ and phase transition enthalpies ΔH can be observed.

This is reflected by a further inventive embodiment, wherein the multicomponent crystal exhibits a phase transition with
- an onset temperature Tₒ as measured by differential scanning calorimetry (DSC) of at least 72 °C,
   and/or
- a peak temperature Tₚ as measured by differential scanning calorimetry (DSC) of at least 75 °C,
   and/or
- a phase transition enthalpy ΔH as measured by differential scanning calorimetry (DSC) of at least 50 J/g,
   preferably
- an onset temperature Tₒ as measured by differential scanning calorimetry (DSC) of at least 85 °C,
   and/or
- a peak temperature Tₚ as measured by differential scanning calorimetry (DSC) of at least 89 °C,
   and/or
- a phase transition enthalpy ΔH as measured by differential scanning calorimetry (DSC) of at least 60 J/g,
   and further preferred
- an onset temperature Tₒ as measured by differential scanning calorimetry (DSC) of at least 98 °C,
   and/or
- a peak temperature Tₚ as measured by differential scanning calorimetry (DSC) of at least 103 °C,
   and/or
- a phase transition enthalpy ΔH as measured by differential scanning calorimetry (DSC) of at least 82 J/g.

Within the context of this disclosure, the onset temperature Tₒ as measured by differential scanning calorimetry (DSC) is defined to be the melting point of the multicomponent crystal or of any other compound to be analyzed, whereas the peak temperature Tₚ as measured by differential scanning calorimetry (DSC) is defined to be the point, where the respective peak in the DSCchart shows its highest or its lowest value.

The highly ordered structure and only a small to no amount of excess or not crystallized active in the inventive multicomponent crystal is also revealed by its high temperature of decomposition T_{d}.

This is reflected by a further embodiment of the inventive multicomponent crystal exhibiting a temperature of decomposition T_{d} as measured by thermogravimetric analysis (TGA), which is at least 175 °C, preferably at least 180 °C, further preferred at least 185 °C, still further preferred at least 190 °C and yet further preferred is 195 °C or higher.

A process for preparing a multicomponent crystal of the invention from at least two solids or from at least one solid and at least one liquid comprises the steps:
i) providing an active, said active being a mixture of at least two molecules of formula (1) 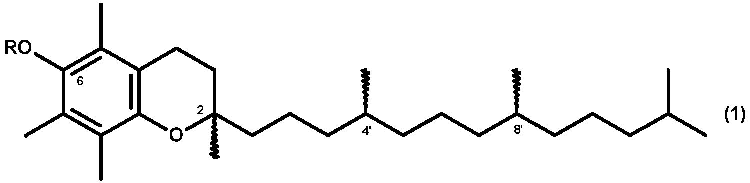
   - with R being at least one selected from the group consisting of H, COCH₃, COCH₂CH₃, CO(CH₂)₁₄CH₃, CO(CH₂)₁₆CH₃,
   - with the methyl groups in position 2, 4', 8' respectively having a S-configuration or a R-configuration,
ii) adding to the active of step i) a crystallizing agent (2), said crystallizing agent (2) exhibiting the property of
   - being suitable to form ions and/or
   - containing a H-bond donor and/or
   - containing a H-bond acceptor and/or
   - being suitable to form a zwitterionic structure and/or
   - having a molecular weight, which ranges from 100 g/mol to 135 g/mol,
iii) mixing the active and the crystallizing agent (2) to obtain a mixture,
iv) optionally concentrating the mixture of step iii) or adding a solvent and/or an antisolvent to the mixture of step iii), while stirring,
v) cooling the mixture of step iii) or of step iv) for crystallization, and/or stirring the mixture of step iii) or of step iv) for crystallization,
vi) incubating the mixture of step v) in order to get the formed multicomponent crystal settled,
vii) removing the supernatant formed in step vi) by decanting or by evaporation or by filtration,
viii) optionally washing the formed multicomponent crystal of step vii) with the solvent and/or with the antisolvent
ix) and drying the formed multicomponent crystal of step vii) or viii),
with the proviso that the combination of proline as crystallizing agent (2) and tocopherol as active is excluded from the teaching of this process.

Said process of obtaining the inventive multicomponent crystal. is straight forward since all process steps can be realized within hours and only exceptionally over-night runs are required. Highly trained stuff is not required since every process step is self explaining. The active and the crystallizing agent (2) not harmful when handled with ordinary lab skills. This further applies when both the active and the crystallizing agent (2) in a preferred embodiment have the GRAS-status, which means they are Generaly Recognized As Save by the FDA. The process provides for using the active and the crystallizing agent (2) as such or in dilution. All process steps can be safely realized and are not harmful as well. It is apparent to the skilled person that the process steps are not expensive either. The way of conducting said process is easy to realize. By said inventive process the double molar amount of active with respect to crystallizing agent (2) and even more can become an integral, viz properly allocated part of the new multicomponent crystal. As already explained supra, non-crystallized matter can be avoided to a large extent or even completely to become a part of the multicomponent crystal. A further advantage of the inventive process is that it provides the inventive multicomponent crystal as fines, which can be directly further processed.

Tocopherol as understood within the phrasing "with the proviso that the combination of proline as crystallizing agent (2) and tocopherol as active is excluded from the teaching of this process." means natural tocopherol and synthetic tocopherol. Natural tocopherol comprises two types of tocopherols and tocotrienols, and comprises 8 kinds of compounds, namely α-, β-, γ-, δ-tocopherols and α-, β-, γ-, δ-tocotrienols. Synthetic tocopherol refers to α-tocopherol, and has 8 optical isomers, viz. at least one, two, three, four, five, six, seven or eight of RRR, RRS, RSS, SSS, RSR, SRS, SRR and SSR.

Proline as understood within the phrasing "with the proviso that the combination of proline as crystallizing agent (2) and tocopherol as active is excluded from the teaching of this process." means proline to comprise D-proline, or L-proline, or a racemic mixture of the both.

A solvent is understood to be any solvent which solubilizes at least the active, but also the active and the crystallizing agent (2). Appropriate solvents are selected form the group consisting of methanol, ethanol, iso-propanol, heptane, acetonitrile and ethyl acetate and mixtures thereof.

An antisolvent is meant to be each solvent, which reduces the amount of solubilized active and/or of solubilized crystallizing agent (2) or completely prevents solubilization of the active and/or of the crystallizing agent (2). An antisolvent is understood to be a solvent that causes precipitation when added to a solution in another solvent. Nitromethane or water is used as antisolvent.

Incubating in step vi) is meant to leave the mixture of step v) with or without stirring at a reduced temperature, preferably at the temperature at which it was cooled in step v).

The supernatant of step vii) is understood to be the liquid which remains above the formed multicomponent crystal of the invention.

As can be seen from the examples below, it is sometimes difficult to work with an active and a crystallizing agent (2), especially, if the amount of active is increased with respect to the amount of crystallizing agent (2). This is fixed with an extension of the inventive process, wherein the active is diluted with or dissolved in an active solubilization solvent prior to providing it in step i) of claim 10.

An active solubilization solvent is a solvent which solubilizes either the active or the crystallizing agent (2) or both of them. The special feature of the active solubilization solvent is, that it is used prior to the process as disclosed in claim 10. Appropriate solvents are selected form the group consisting of methanol, ethanol, iso-propanol, heptane, acetonitrile and ethyl acetate and mixtures thereof.

A further developed embodiment of the inventive process provides
- the active being a mixture of at least three molecules of formula (1), preferably of at least four molecules of formula (1), further preferred of at least five molecules of formula (1), yet further preferred of at least six molecules of formula (1), still further preferred of at least seven molecules of formula (1) and mostly preferred of at least eight molecules of formula (1),
   and/or
- R being at least one selected from the group consisting of H and COCH₃,
   and/or
- the crystallizing agent (2) being betaine of formula (2) 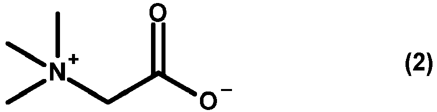 in its ionic form, which it adopts during crystallization.

Upon using the process features given supra, multicomponent crystals of high stability are obtained.

This is even further pronounced with the following advancement of the inventive process, which defines
- said active being D/L-α-tocopherol of formula (3) 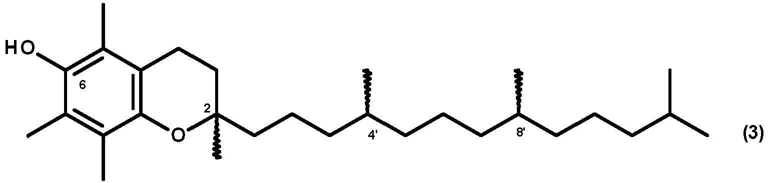 with the methyl groups in position 2, 4', 8' respectively having a S-configuration or a R-configuration,
   and/or
- the crystallizing agent (2) being betaine of formula (2) 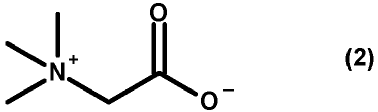 in its ionic form, which it adopts during crystallization.

The multi-component crystal according to the invention in one alternative may be used directly in the form of powders, granules, suspensions.

In another alternative it may be combined with other dietary or pharmaceutically acceptable ingredients by admixing the multicomponent crystal and the ingredients to obtain a mixture and optionally finely grinding the components of said mixture, and then filling them into capsules. Said capsules may be composed for example of hard or soft gelatin.

Part of the invention is also a composition comprising the multicomponent crystal as defined in at least one of the embodiments mentioned supra or as obtained in at least one of the process embodiments as previously defined and at least one dietary acceptable constituent.

Said composition in one embodiment is a dietary supplement.

In another embodiment it is a pharmaceutical composition, preferably a pharmaceutical composition further comprising at least one pharmaceutically acceptable carrier, and/or at least one diluent, and/or further ingredients, and/or at least one pharmaceutical excipient.

The composition in one extension is a pre-formulation, an oral formulation, a solid formulation such as a powder, a capsule, a tablet, a pill, a troche or a liquid suspension formulation.

A further important aspect of the invention is the use of a multicomponent crystal as previously defined or as obtained in a process as identified supra, or the use of the above disclosed composition in a food formulation or a feed formulation or in a pharmaceutical preparation.

In the following, the present invention will be further illustrated by particular embodiments as disclosed in the examples and with reference made to figures. However, said detailed description and examples are not understood to narrow the gist of the invention down thereto. They merely serve to illustrate the invention in more details.

### Abbreviations

- HPLC: high pressure liquid chromatography
- ATR-IR: attenuated total reflectance infrared spectroscopy
- MeOH: methanol
- NMR: nuclear magnetic resonance
- TG: thermogravimetry
- r.h.: relative humidity (air, if not indicated otherwise)
- w%: weight%
- v/v: volume by volume
- PXRD: powder X-ray diffraction
- DSC: differential scanning calorimetry

### Figures:

- Fig. 1:: PXRD pattern of a all-rac α-tocopherol - betaine co-crystal from crystallization in MeOH with the molar ratio of all-rac-α-tocopherol to betaine being 2:1; Cu Kα radiation.
- Fig. 2:: TG data of a all-rac α-tocopherol - betaine co-crystal from crystallization in MeOH with the molar ratio of all-rac-α-tocopherol to betaine being 2:1, heating rate 10°C/min.
- Fig. 3:: DSC data of a all-rac α-tocopherol - betaine co-crystal from crystallization in MeOH with the molar ratio of all-rac α-tocopherol to betaine being 2:1, heating rate 10°C/min, recording of the exothermal phase transition during heating up.
- Fig. 4:: PXRD pattern of all-rac α-tocopherol - betaine co-crystal from crystallization in MeOH with the molar ratio of all-rac α-tocopherol to betaine being 2:1; Cu Kα radiation.
- Fig. 5:: PXRD pattern of all-rac α-tocopherol - betaine co-crystal from crystallization in MeOH with the molar ratio of all-rac α-tocopherol to betaine being 2:1; Cu Kα radiation.
- Fig. 6:: ATR-IR spectrum of all-rac-α-tocopherol - betaine co-crystal from crystallization in MeOH with the molar ratio of all-rac α-tocopherol to betaine being 2:1.
- Fig. 7:: HPLC data of all-rac α-tocopherol - betaine co-crystal from crystallization in MeOH with the molar ratio of all-rac α-tocopherol to betaine being 2:1, 295 nm.

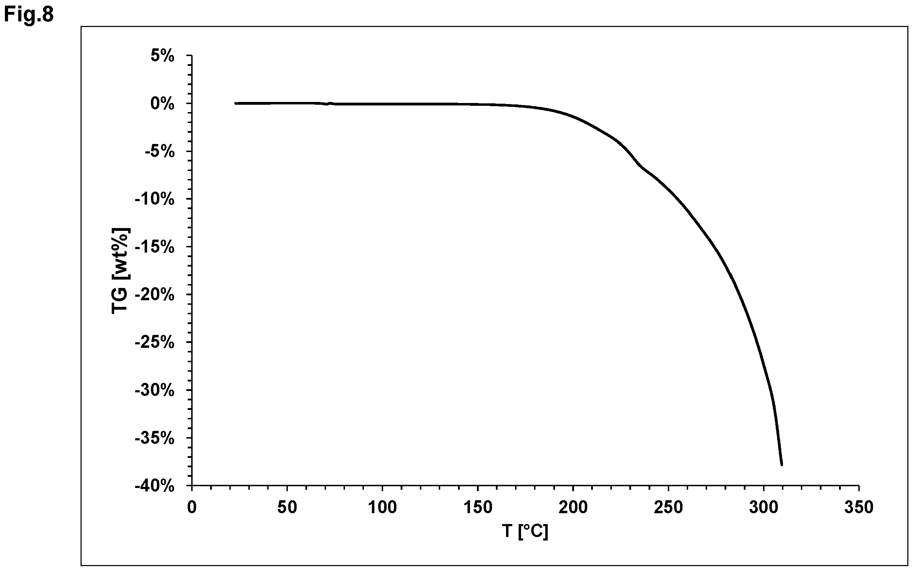

- Fig. 8:: TG data of all-rac α-tocopherol - L-proline co-crystal from crystallization in MeOH with the molar ratio of all-rac α-tocopherol to proline being 2:1, heating rate 10°C/min.

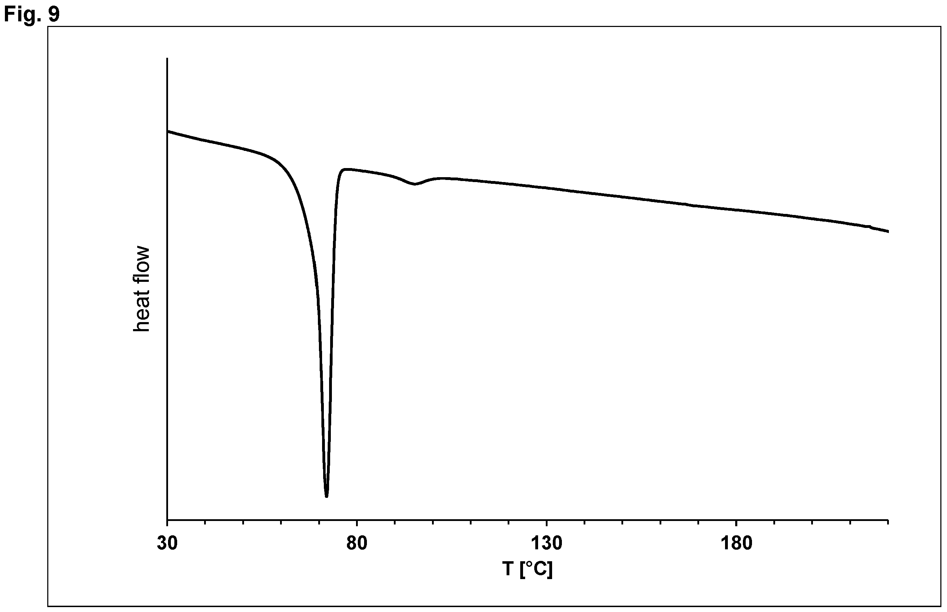

- Fig. 9:: DSC data of all-rac α-tocopherol - L-proline co-crystal from crystallization in MeOH with the molar ratio of all-rac α-tocopherol to proline being 2:1, heating rate 10°C/min, recording of the exothermal phase transition during heating up.
- Fig. 10:: PXRD pattern of all-rac α-tocopherol - L-proline co-crystal from crystallization in MeOH : nitromethane (1:1 v/v) with the molar ratio of all-rac α-tocopherol to proline being 1:1; Cu Kα radiation.
- Fig. 11:: PXRD pattern of D-α-tocopherol - betaine co-crystal from crystallization in MeOH with the molar ratio of D-α-tocopherol to betaine being 2:1; Cu Kα radiation.
- Fig. 12:: PXRD pattern of all-rac α-tocopherol - betaine co-crystal 2:1 (bottom, example 1) in comparison to D-α-tocopherol - betaine co-crystal from example 13 (top; counts + 500); Cu Kα radiation.

### Examples

Wherever noted, in the following, room temperature depicts a temperature from the range 22-25 °C, ambient temperature is defined as 25±10 °C and percentages are given by weight, if not indicated otherwise.

### Instrumental:

### Powder X-ray diffraction:

The measurements were carried out with a Panalytical X'Pert Pro diffractometer (manufacturer: Panalytical) using Cu Kα radiation in the Bragg-Brentano reflection geometry. Generally, the 2θ values are accurate within an error of ±0.1-0.2°. The relative peak intensities can vary considerably for different samples of the same crystalline form because of different preferred orientations of the crystals. The samples were prepared without any special treatment other than the application of slight pressure to get a flat surface. Generally, silicon single crystal sample holders of 0.2 mm depth were used. The tube voltage and current were 45 kV and 40 mA, respectively. Diffraction patterns were recorded in the range from 2θ =3°-40° with increments of 0.0167°. The samples were rotated during the measurement.

### Thermogravimetry:

TGA data were recorded with a TG/DTA 7200 (SII Nano Technology Inc). The samples were placed in platinum standard pans. The sample size in each case was 2 to 10 mg. The heating rate was 10°C/min. The samples were purged with a stream of synthetic air during the experiment.

### Differential scanning calorimetry (DSC):

DSC data were recorded with a Mettler Toledo DSC 823e/700/229 module. The samples were placed in aluminum standard pans. The sample size in each case was 1 to 10 mg. The heating rate was 10°C/min. The samples were purged with a stream of nitrogen during the experiment. The onset point of the endothermic event is reported as melting point.

### ¹H-NMR:

The ¹H-NMR spectra were recorded on a Bruker AVN-600 spectrometer using deuterated solvents.

### ATR-IR spectroscopy:

ATR-IR spectra were measured at room temperature on Nicolet iS50_2. 32 scans with a resolution of 4 cm⁻¹ measured in the range 4000 cm⁻¹ to 400 cm⁻¹.

### HPLC:

Approximately 10 mg solid material was dissolved in approx. 8 g of isopropanol. The solution was directly used for analysis by HPLC on a Agilent 1200, equipped with a DAD1 UV detector (evaluation at 295 nm) and Sampler Type G1313A. Chromeleon 7.2.8 software was used to record the chromatograms and to calculate the chromatographic parameters. Gradient elution (solvent A: ACN + 0.1% formic acid / solvent B: water + 0.1% formic acid, gradient see below) was achieved using CHIRALPAK IG-3, 150*2.1mm, 3µm, Daicel. Injection volume was set to 5 µL by auto injector. The analysis was performed with a flux rate of 0.5 ml/min.

### Gradient

| time | % solvent A | % solvent B |
|---|---|---|
| 0.0 | 45 | 55 |
| 23.0 | 30 | 70 |
| 35.0 | 20 | 80 |
| 35.1 | 0 | 100 |
| 40.0 | 0 | 100 |
| 40.1 | 45 | 55 |
| 51.0 | 45 | 55 |

Solvents: For all experiments, standard grade solvents are used.

### Examples:

### Example 1:

### all-rac α-Tocopherol - Betaine Co-Crystal 2:1

The all-rac -α-tocopherol - betaine co-crystal 2:1 is prepared from all-rac α-tocopherol and betaine.

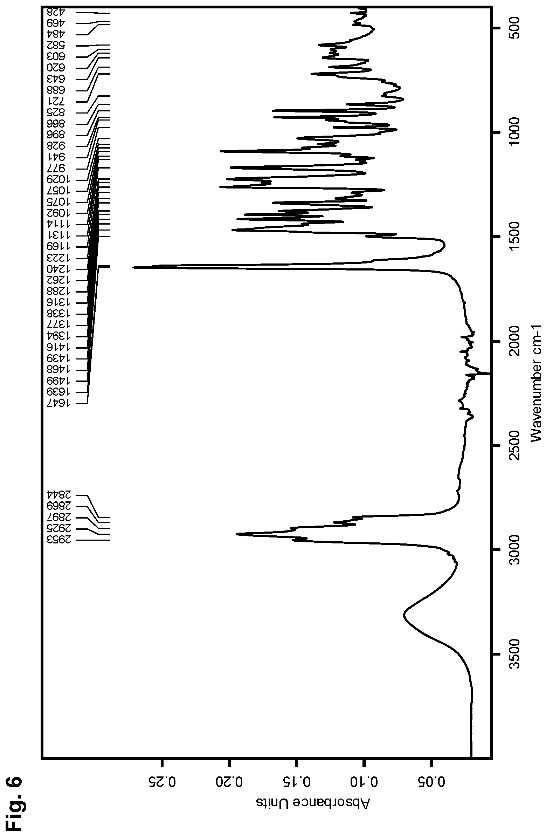

The PXRD pattern is displayed in Figure 1. Characteristic PXRD peaks (expressed in °2θ ± 0.2 °2θ; Cu Kα radiation) are observed at 3.7, 5.5, 7.4, 9.2, 11.1, 12.9, 14.8, 15.6, 16.7, 17.0, 18.1, 18.5, 20.4, 21.8 and 24.2. The ATR-IR data is presented in Fig. 6. Characteristic peaks are observed at 2953, 2897, 1647, 1639, 1223, 1029 and 721 cm⁻¹.

### Example 1a:

431 mg all-rac α-tocopherol (1 mmol) and 117 mg betaine (1 mmol) (ratio 1:1) were dissolved in 4 mL MeOH at 60°C. After 0.5 h the solution was cooled down to -10°C (cooling rate -5°C/h, stirring rate 120 rpm). The precipitated multicomponent crystal was filtered and dried in vacuum at room temperature. The solid material shows a PXRD pattern as in Figure 1 having characteristic PXRD peaks (expressed in °2θ ± 0.2 °2θ; Cu Kα radiation) as indicated in example 1. ¹H-NMR spectroscopy indicates a molar ratio of all-rac α-tocopherol to betaine of about 2:1. TG data show no significant weight loss up to the melting peak and above, first significant weight loss is observed around 200°C. DSC data show a melting point with an onset of 98°C and a peak maximum of 103°C as well as a transition enthalpy ΔH of 82 J/g.

Said transition enthalpy is almost the double of the value obtained for an all-rac α-tocophero-TOC - L-proline co-crystal as indicated in comparative example 8. Likewise, the onset of 98 °C and the peak maximum of 103 °C is about 30 °C higher than this one obtained for the co-crystal of comparative example 8. The significant weight loss starts at about 200 °C which is about 30 °C higher than the value obtained for a said all-rac α-tocophero - L-proline co-crystal as indicated in comparative example 8. These observations prove that much more energy is required to disassemble the multicomponent crystal of example 1a, which is only possible if said crystal is much more structured and thus has a higher crystallinity compared to the co-crystal of comparative example 8.

### Example 2:

431 mg all-rac α-tocopherol (1 mmol) and 117 mg betaine (1 mmol) (ratio 1:1) were dissolved in 4 mL MeOH at 60°C. After 0.5 h the solution was cooled down to -10°C (cooling rate -5°C/h, stirring rate 120 rpm). The precipitated multicomponent crystal was filtered and dried in vacuum at room temperature to yield 303 mg all-rac α-tocopherol - betaine co-crystal. The PXRD pattern is similar to the pattern presented in Figure 1.

### Example 3:

1725 mg all-rac α-tocopherol (4 mmol) and 235 mg betaine (2 mmol) (ratio 2:1) were dissolved in 4 mL MeOH at 60°C. After 0.5 h the solution was cooled down to -10°C (cooling rate -5°C/h, stirring rate 120 rpm). The precipitated multicomponent crystal was filtered and dried in vacuum at room temperature to yield 1093 mg all-rac α-tocopherol - betaine co-crystal. The PXRD pattern is similar to the pattern presented in Figure 1.

### Example 4:

6.9 g all-rac α-tocophero (16 mmol)l and 0.94 g betaine (8 mmol) (ratio 2:1) were dissolved in 16 mL MeOH at 60°C. After 0.5 h the solution was cooled down to -10°C (cooling rate -5°C/h, stirring rate 200 rpm). The precipitated multicomponent crystal was filtered and dried in vacuum at room temperaturα to yield 5.0 mg all-rac α-tocopherol - betaine co-crystal. The PXRD pattern is similar to the pattern presented in Figure 1. The DSC data shows a melting point with an onset of 98°C and a peak temperature of 104°C as well as an enthalpy ΔH of 70 J/g.

### Example 5:

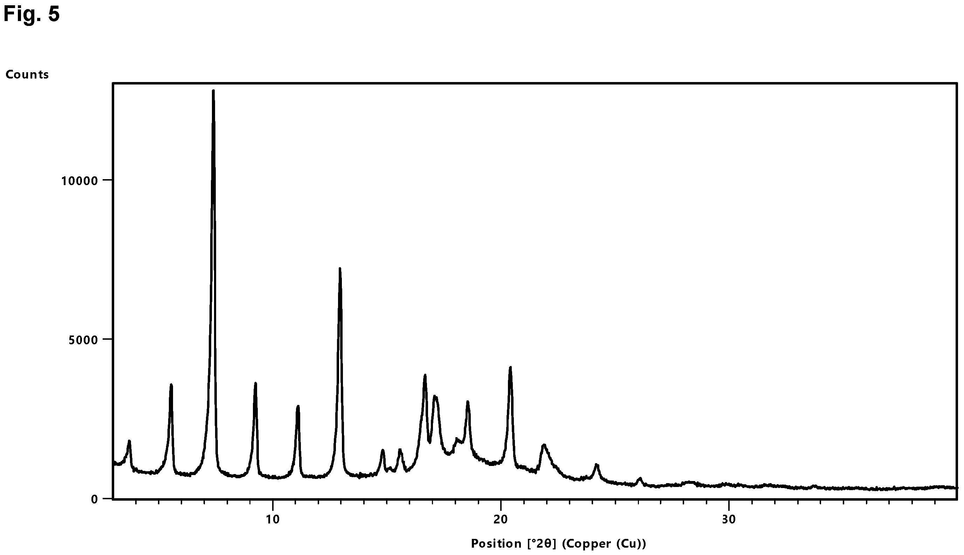

6.9 g all-rac α-tocopherol (16 mmol) and 0.94 g betaine (8 mmol) (ratio 2:1) were dissolved in 16 mL MeOH at 60°C. After 0.5 h the solution was cooled down to -10°C (cooling rate -5°C/h, stirring rate 1200 rpm). The precipitated multicomponent crystal was filtered, washed with 5 mL methanol and dried in vacuum at room temperature, yield 4,4 g all-rac α-tocopherol - betaine co-crystal. The PXRD pattern is similar to the pattern presented in Figure 5. The DSC data shows a melting point with an onset of 100°C and a peak temperature of 104°C (72 J/g). The ATR-IR spectrum is displayed in Figure 6. The result of the HPLC measurement is shown Fig 7. The chiral HPLC method does not separate alle eight isomers, but the ratio of 1 (12.12 area%) : 3 (37.57 ar-ea%) : 1 (12.74 area%) : 2 (25.16 are%) : 1 (12.41 area%) is the same as in all-rac α-tocopherol (1 (12.18 area%) : 3 (37.81 area%) : 1 (12.66 area%) : 2 (25.11 area%) : 1 (12.25 area%)) (Figure 7). The data confirm that all eight all-rac α-tocopherol isomers are incorporated into the co-crystal.

### Example 6:

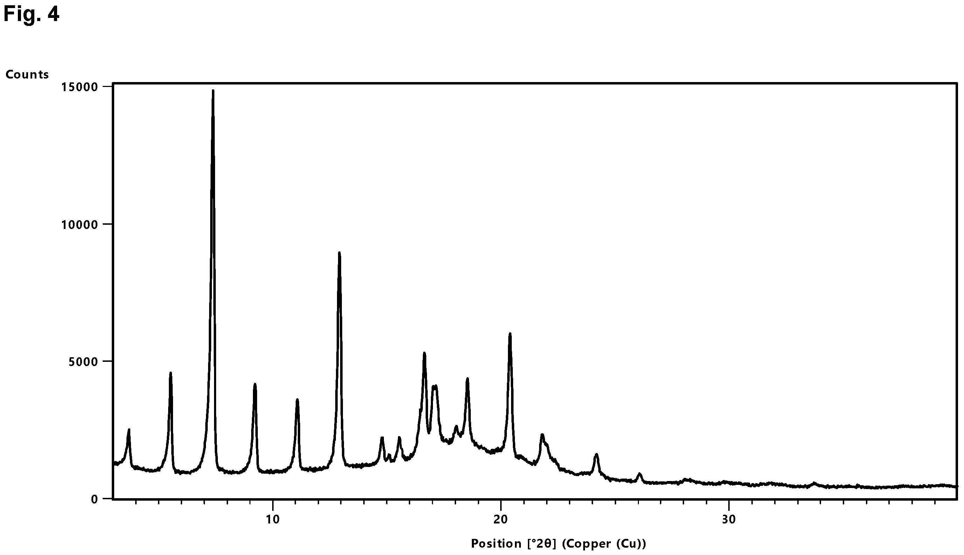

6.9 g all-rac α-tocopherol (16 mmol) and 0.94 g betaine (8 mmol) (ratio 2:1) were dissolved in 16 mL MeOH at 60°C. After 0.5 h the solution was cooled down to -10°C (cooling rate -5°C/h, stirring rate 1200 rpm). The precipitated multicomponent crystal was filtered, washed with 5 mL nitromethane and dried in vacuum at room temperature to yield 4.8 g all-rac α-tocopherol - betaine co-crystal. The solid material shows a PXRD pattern as in Figure 4. The DSC data shows a melting point with an onset of 101°C and a peak temperature of 103°C as well as an enthalpy ΔH of 60 J/g.

### Example 7:

6.9 g all-rac α-tocopherol and 0.94 g betaine (ratio 2:1) was dissolved in 16 mL MeOH at 60°C. After 0.5 h the solution was cooled down to -10°C (cooling rate -5°C/h, stirring rate 1200 rpm). The precipitated was filtered, washed with 5 mL heptane and dried in vacu-um at room temperature, yield 2,8 mg all-rac α-tocopherol - betaine co-crystal. The PXRD pattern is similar to the pattern presented in Figure 1. The DSC data shows a melting point with an onset of 101°C and a peak temperature of 105°C as well as an enthalpy ΔH of 81 J/g.

### Example 7b:

139 g all-rac α-tocopherol (323 mmol) and 18 g betaine (154 mmol) (ratio 2:1)(were added to a 250 mL glass tall-form beaker cooled via an ice bath. The mixture was blended using a Polytron PT3100 D high shear mix-er at 30000 rpm for 40 seconds fitted with a rotor-stator-head. Afterwards, the mixture was stirred via an IKA Eurostar 20 stirrer fitted with a dissolver. The temperature was kept be-low 50°C. The mixture was agitated for 2.25 h. After 50 minutes, the stirrer speed was stepwise reduced from the initial 5000 rpm because of the increasing viscosity. After 1 h and 23 min, the stirrer speed was kept at 1000 rpm. Starting 30 minutes after the beginning of the agitation, periodically over the course of the experiment, the material was manually homogenized as the dissolver was not able to effectively agitate the complete reaction mixture. The PXRD pattern is similar to the pattern presented in Figure 1.

### Comparative Experiments

All-rac α-tocophero - L-proline co-crystal is described in WO 2019/128175 (corresponding to EP 3733657 A1) with PXRD patterns of lowest crystallinity and amorphous content. Following examples are modifies examples from said WO 2019/128175.

### Example 8:

431 mg all-rac α-tocopherol (1 mmol)) and 115 mg L-proline (1 mmol) (ratio 1:1) were dissolved in 4 mL methanol : nitro-methane (1:1) at 60°C. After 1 h the solution was cooled down to -10°C (cooling rate -5°C/h, stirring rate 120 rpm). The precipitated co-crystal was filtered and dried in vacuum at room temperature and all-rac α-tocopherol - L-proline co-crystal was obtained. The solid material shows a very poor PXRD pattern similar to Fig ity 10.

¹H-NMR spectroscopy indicates a molar ratio of all-rac α-tocopherol to L-proline of about 2:1. TG data show no significant weight loss up to the melting peak and above, first significant weight loss is observed around 170°C. DSC data show a melting point with an onset of 69°C and 72°C peak maximum (44 J/g).

These values being low compared to those of example one, show a lower stability of the co-crystal obtained in comparative example 9.I

### Example 9:

473 mg all-rac α-tocopherol I (1,1 mmol) and 127 mg L-proline (1,1 mmol) (ratio 1:1) were dissolved in 4 mL methanol : nitro-methane (1:1) at 60°C. After 1 h the solution was cooled down to -10°C (cooling rate -5°C/h, stirring rate 120 rpm). The precipitated co-crystal was filtered and dried in vacuum at room temperature to yield ∼150 mg all-rac α-tocophero - L-proline co-crystal. The solid material shows a PXRD pattern as in Figure 10.

### Example 10:

529 mg all-rac α-tocopherol (1,2 mmol) and 71 mg L-proline (0,6 mmol) (ratio 2:1) was dissolved in 4 mL methanol : nitrome-thane (1:1) at 60°C. After 1 h the solution was cooled down to -10°C (cooling rate -5°C/h, stirring rate 120 rpm). The precipitated was filtered and dried in vacuum at room temperature, yield ~100 mg all-rac α-tocopherol - L-proline co-crystal.The solid material shows a PXRD pattern as in Figure 10.

### Example 11:

529 mg all-rac α-tocopherol (1,2 mmol) and 71 mg L-proline (0,6 mmol) (ratio 2:1) was dissolved in 4 mL MeOH at 60°C. After 1 h the solution was cooled down to -10°C (cooling rate - 5°C/h, stirring rate 120 rpm). The precipitated co-crystal was filtered and dried in vacuum at room temperature, yield 9 mg all-rac α-tocophero - L-proline co-crystal, characterization PXRD.The solid material shows a PXRD pattern as in Figure 10.

### Example 12:

473 mg all-rac α-tocopherol (1,1 mmol) and 127 mg L-proline (1,1 mmol) (ratio 1:1) were dissolved in 4 mL MeOH at 60°C. After 1 h the solution was cooled down to -10°C (cooling rate - 5°C/h, stirring rate 120 rpm). The precipitated co-crystal was filtered and dried in vacuum at room temperature to yield 89 mg all-rac α-tocopherol - L-proline co-crystal. The solid material shows a PXRD pattern as in Figure 10.

A D-α-tocopherol - betaine co-crystal was described in Int. J. Pharm. 592 (2021) 120057. The experiment was reproduced as it was possible. No dissolution at room temperature was observed. Therefore, the solid was dissolved at 60°C, before cooling was started.

### Example 13:

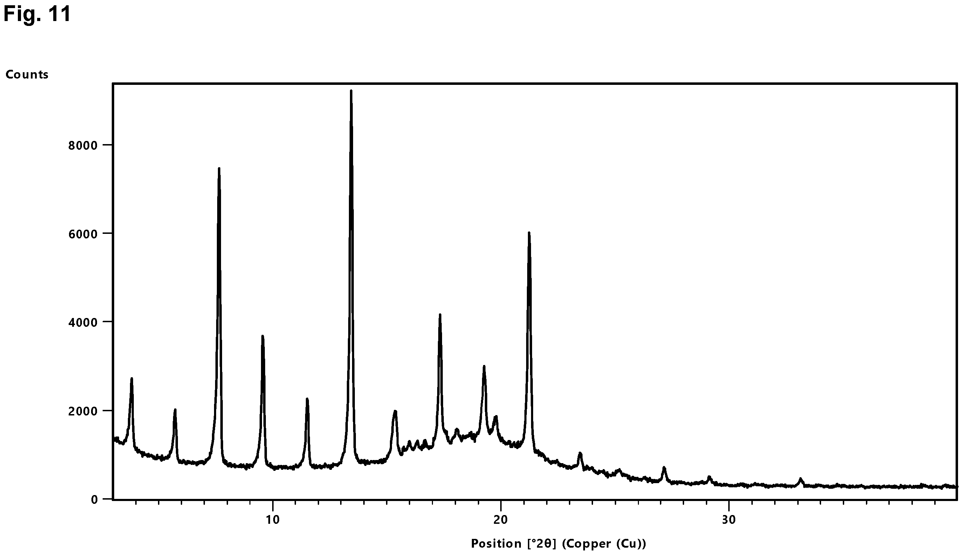

4.3 g d-αToc (10 mmol) and 0.585 g betaine (5 mmol) (ratio 2:1) were dissolved in 10 mL methanol at 60°C and the solution was cooled at -20°C (cooling rate -5°C/h, stirring rate 1200 rpm). The powder was filtered and dried in vacuum at room temperature. The solid material shows a PXRD pattern as in Figure 11.

The PXRD pattern of all-rac α-tocopherol - betaine multicomponent crystal 2:1 (bottom chart, from example 1) was compared with the D-α-tocopherol - betaine co-crystal (upper chart, from of example 13) by superposition in Figure 12.

Two things are apparent: First the bottom chart shows different signals, which are shifted to lower 2θ-values when comparing with the 2θ-values of the upper chart. In addition, new signals appear in the bottom chart whereas others disappear, indicating another type of crystal to be formed. Second the bottom chart looks much more homogenous and does not exhibit a large background when comparing with the upper chart.

However, multicomponent crystal formation from all-rac α-tocopherol and betaine is more than surprising taking into account, that all-rac α-tocopherol in the multicomponent crystal of the bottom chart is a mixture of many isomers, whereas D-α-tocopherol in the co-crystal of the upper chart is only one molecule much easier to crystalize.

## Claims

1. Multicomponent crystal comprising
a) an active, said active being a mixture of at least two molecules of formula (1) 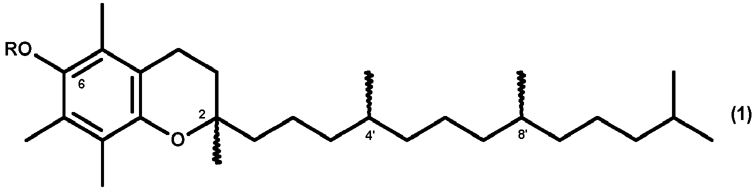 with R being at least one selected from the group consisting of H, COCH₃, COCH₂CH₃, CO(CH₂)₁₄CH₃, CO(CH₂)₁₆CH₃,
- with the methyl groups in position 2, 4', 8' respectively having a S-configuration or a R-configuration,
and
b) a crystallizing agent (2), said crystallizing agent (2) exhibiting the property of
- being suitable to form ions and/or
- containing a H-bond donor and/or
- containing a H-bond acceptor and/or
- being suitable to form a zwitterionic structure and/or
- having a molecular weight, which ranges from 100 g/mol to 135 g/mol,
with the proviso that the combination of proline as crystallizing agent (2) and tocopherol as active is excluded from the teaching of claim 1.

2. The multicomponent crystal of claim 1, **wherein**
- said active being a mixture of at least three molecules of formula (1), preferably of at least four molecules of formula (1), further preferred of at least five molecules of formula (1), yet further preferred of at least six molecules of formula (1), still further preferred of at least seven molecules of formula (1) and mostly preferred of at least eight molecules of formula (1),
and/or
- R being at least one selected from the group consisting of H and COCH₃,
and/or
- the crystallizing agent (2) being betaine of formula (2) 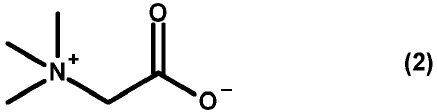 in its ionic form, which it adopts during crystallization.

3. The multicomponent crystal according to claim 1 or 2, **wherein**
- said active being D/L-α-tocopherol of formula (3) 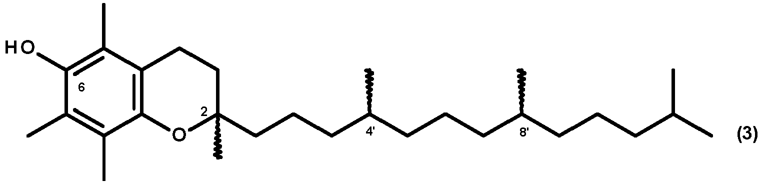 with the methyl groups in position 2, 4', 8' respectively having a S-configuration or a R-configuration,
and/or
- the crystallizing agent (2) being betaine of formula (2) 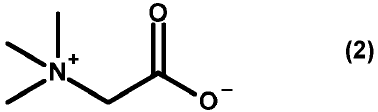 in its ionic form, which it adopts during crystallization.

4. The multicomponent crystal according to any one of claims 1 to 3, **wherein**
the molar ratio of said active to said crystallizing agent (2) ranges from 3:1 to 1:3, preferably from 2.1:1 to 1.9:1 or from 2:1 to 1:2 including a molar ratio of 1:1 and further preferred being 2:1.

5. The multicomponent crystal according to any one of claims 1 to 4, **wherein** it has a powder x-ray diffraction pattern (PXRD pattern) with at least one characteristic peak expressed in °2θ ± 0.2 °2θ (CuKα radiation), which is selected from the peaks located at 5.5, 7.4, 9.2, 12.9, 16.7 and 20.4,
preferably
it has a powder x-ray diffraction pattern (PXRD pattern) with at least three characteristic peaks expressed in °2θ ± 0.2 °2θ (CuKa radiation), which are selected from the peaks located at 5.5, 7.4, 9.2, 12.9, 16.7 and 20.4,
further preferred
it has a powder x-ray diffraction pattern (PXRD pattern) with the following characteristic peaks expressed in °2θ ± 0.2 °2θ (CuKa radiation) and located at 5.5, 7.4, 9.2, 12.9, 16.7 and 20.4.

6. The multicomponent crystal according to any one of claims 1 to 4, **wherein** it has a powder x-ray diffraction pattern (PXRD pattern) with at least one characteristic peak expressed in °2θ ± 0.2 °2θ (CuKa radiation), which is selected from the peaks located at 3.7, 5.5, 7.4, 9.2, 11.1, 12.9, 14.8, 15.6, 16.7, 17.0, 18.1, 18.5, 20.4, 21.8 and 24.2,
preferably
it has a powder x-ray diffraction pattern (PXRD pattern) with at least three characteristic peaks expressed in °2θ ± 0.2 °2θ (CuKα radiation), which are selected from the peaks located at 3.7, 5.5, 7.4, 9.2, 11.1, 12.9, 14.8, 15.6, 16.7, 17.0, 18.1, 18.5, 20.4, 21,8 and 24.2, further preferred
it has a powder x-ray diffraction pattern (PXRD pattern) with the following characteristic peaks expressed in °2θ ± 0.2 °2θ (CuKα radiation) and located at 3.7, 5.5, 7.4, 9.2, 11.1 12.9, 14.8, 15.6, 16.7, 17.0, 18.1, 18.5, 20.4, 21.8 and 24.2.

7. The multicomponent crystal according to any one of claims 1 to 6, **wherein** it comprises less than 45 w% of not crystallized matter, preferably less than 30 w%, further preferred less than 20 w%, still further preferred less than 10 w% and highly preferred less than 5 w% including 1 w% and 0 w%.

8. The multicomponent crystal according to any one of claims 1 to 7, **wherein** it exhibits a phase transition with
- an onset temperature Tₒ as measured by differential scanning calorimetry (DSC) of at least 72 °C,
and/or
- a peak temperature Tₚ as measured by differential scanning calorimetry (DSC) of at least 75 °C,
and/or
- a phase transition enthalpy ΔH as measured by differential scanning calorimetry (DSC) of at least 50 J/g,
preferably
- an onset temperature Tₒ as measured by differential scanning calorimetry (DSC) of at least 85 °C,
and/or
- a peak temperature Tₚ as measured by differential scanning calorimetry (DSC) of at least 89 °C,
and/or
- a phase transition enthalpy ΔH as measured by differential scanning calorimetry (DSC) of at least 60 J/g,
and further preferred
- an onset temperature Tₒ as measured by differential scanning calorimetry (DSC) of at least 98 °C,
and/or
- a peak temperature Tₚ as measured by differential scanning calorimetry (DSC) of at least 103 °C,
and/or
- a phase transition enthalpy ΔH as measured by differential scanning calorimetry (DSC) of at least 82 J/g.

9. The multicomponent crystal according to any one of claims 1 to 8, **wherein** its temperature of decomposition T_{d} as measured by thermogravimetric analysis (TGA) is at least 175 °C, preferably at least 180 °C, further preferred at least 185 °C, still further preferred at least 190 °C and yet further preferred is 195 °C or higher.

10. Process for preparing a multicomponent crystal as defined in any one of claims 1 to 9 from at least two solids or from at least one solid and at least one liquid comprising the steps:
i) providing an active, said active being a mixture of at least two molecules of formula (1) 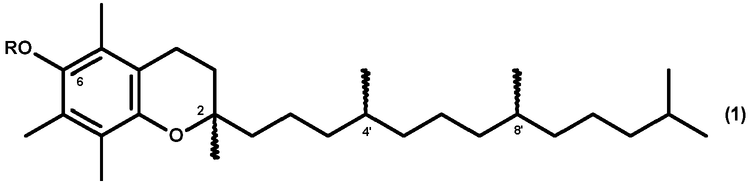
- with R being at least one selected from the group consisting of H, COCH₃, COCH₂CH₃, CO(CH₂)₁₄CH₃, CO(CH₂)₁₆CH₃,
- with the methyl groups in position 2, 4', 8' respectively having a S-configuration or a R-configuration,
ii) adding to the active of step i) a crystallizing agent (2), said crystallizing agent (2) exhibiting the property of
- being suitable to form ions and/or
- containing a H-bond donor and/or
- containing a H-bond acceptor and/or
- being suitable to form a zwitterionic structure and/or
- having a molecular weight, which ranges from 100 g/mol to 135 g/mol,
iii) mixing the active and the crystallizing agent (2) to obtain a mixture,
iv) optionally concentrating the mixture of step iii) or adding a solvent and/or an antisolvent to the mixture of step iii), while stirring,
v) cooling the mixture of step iii) or of step iv) for crystallization, and/or stirring the mixture of step iii) or of step iv) for crystallization,
vi) incubating the mixture of step v) in order to get the formed multicomponent crystal settled,
vii) removing the supernatant formed in step vi) by decanting or by evaporation or by filtration,
viii) optionally washing the formed multicomponent crystal of step vii) with the solvent and/or with the antisolvent
ix) and drying the formed multicomponent crystal of step vii) or viii),
with the proviso that the combination of proline as crystallizing agent (2) and tocopherol as active is excluded from the teaching of claim 10.

11. The process according to claim 10, **wherein** the active is diluted with or dissolved in an active solubilization solvent prior to providing it in step i) of claim 10.

12. The process according to claim 10 or 11, **wherein**
- the active being a mixture of at least three molecules of formula (1), preferably of at least four molecules of formula (1), further preferred of at least five molecules of formula (1), yet further preferred of at least six molecules of formula (1), still further preferred of at least seven molecules of formula (1) and mostly preferred of at least eight molecules of formula (1),
and/or
- R being at least one selected from the group consisting of H and COCH₃,
and/or
- the crystallizing agent (2) being betaine of formula (2) 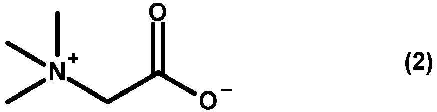 in its ionic form, which it adopts during crystallization.

13. The process according to any one of claims 10 to 12, **wherein**
- said active being D/L-α-tocopherol of formula (3) 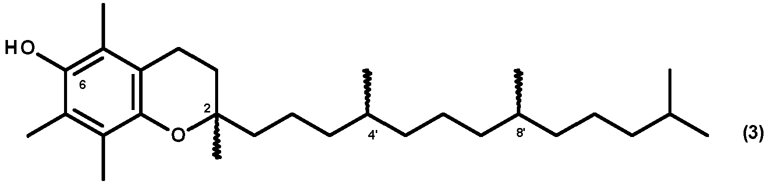 with the methyl groups in position 2, 4', 8' respectively having a S-configuration or a R-configuration,
and/or
- the crystallizing agent (2) being betaine of formula (2) 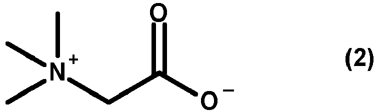 in its ionic form, which it adopts during crystallization.

14. A composition comprising a multicomponent crystal as defined in any one of claims 1 to 9 or as obtained in a process according to any one of claims 10 to 13 and at least one dietary acceptable constituent.

15. Use of a multicomponent crystal as defined in any one of claims 1 to 9 or as obtained in a process according to any one of claims 10 to 13, or of a composition according to claim 14 in a food formulation or a feed formulation or in a pharmaceutical preparation.
